# FASCICULE DE BREVET EUROPEEN

(11) **EP 0 696 210 B1**
(45) Date de publication et mention de la délivrance du brevet: **02.09.1998**
(21) Numéro de dépôt: 94914430.7
(22) Date de dépôt: 21.04.1994
(51) Int. Cl.: A61L 27/00, A61F 2/12

(54) **UTILISATION D'HYDROGELS A BASE D'ACIDE HYALURONIQUE ET/OU DE POLYDESOXYRIBONUCLEOTIDES COMME MATERIAUX DE REMPLISSAGE DE PROTHESES ET PROTHESES EN RESULTANT**
VERWENDUNG VON HYDROGELEN AUF DER BASIS VON HYALURONSÄURE UND/ODER POLYDEOXYRIBONUKLEOTIDEN ALS FÜLLMATERIAL FÜR PROTHESEN UND SO ERHALTENE PROTHESEN
USE OF HYALURONIC ACID AND/OR POLYDEOXYRIBONUCLEOTIDE BASED HYDROGELS AS MATERIALS FOR FILLING PROSTHESES AND PROSTHESES OBTAINED

(30) Priorité: 30.04.1993 FR 9305135
(43) Date de publication de la demande: 14.02.1996
(73) Titulaire: LABORATOIRES SEBBIN, 95300 Pontoise (FR)
(72) Inventeur: LE PESTEUR, Jacques, F-75006 Paris (FR); CHITARRINI, Gino, U., F-95100 Argenteuil (FR)
(74) Mandataire: Giraud, Françoise
(86) Numéro de dépôt international: FR9400448
(87) Numéro de publication internationale: WO9425078

(56) Documents cités:
- EP-A- 0 466 300
- US-A- 5 219 360

## Description

La présente invention concerne l'utilisation d'hydrogels à base d'acide hyaluronique et/ou de polydésoxyribonucléotides comme matériaux de remplissage de prothèses, en particulier de prothèses mammaires et les prothèses en résultant.

L'implantation de prothèses en vue de remplacer ou remodeler certains tissus mous est une technique chirurgicale maintenant devenue relativement courante.

Une telle technique est particulièrement utilisée dans la chirurgie des seins où il existe deux types de prothèses mammaires :
- celles dites "pré-remplies", constituées d'une poche en élastomère de silicone remplie de gel de silicone, mises au point en 1962 par le Docteur CRONIN à HOUSTON (U.S.A.).
- celles dites "gonflables", constituées d'une poche en élastomère de silicone comprenant une valve permettant l'introduction de :
   . sérum physiologique répondant à la pharmacopée française ayant une concentration en sel de sodium de (9 %).
   . polyvinylpyrrolidone ou P.V.P, substance maniable et de haut poids moléculaire.

On a également signalé récemment l'existence d'une prothèse mammaire remplie d'un mélange constitué de 6 % de polysaccharides et de 94 % de sérum physiologique et mise sur le marché sous le nom d'Hydrogel par la société P.I.P.

Il existe à l'heure actuelle une vive polémique concernant l'utilisation des prothèses remplies de gel de silicone, due à une suspicion d'éventuelle relation entre l'utilisation de gel de silicone et l'apparition de maladies auto-immunes.

Par ailleurs, les autres matériaux actuellement utilisés pour le remplissage des implants ne présentent pas à l'heure actuelle tous les avantages que l'on pourrait attendre d'un matériau destiné à remplacer un tissu animal ou humain.

En effet, lorsque l'enveloppe est remplie de sérum physiologique ou d'un autre élément liquide, on obtient une homothéticité insuffisante entre l'enveloppe et son contenu. Par ailleurs, le matériau de remplissage manque de souplesse, du fait du caractère incompressible de l'eau.

L'acide hyaluronique et ses sels sont des produits utilisables comme produits injectables. Par ailleurs, les gels d'acide hyaluronique sont utilisés dans le domaine de la chirurgie, en particulier, en ophtalmologie.

On a signalé également plus récemment dans le Quotidien du Médecin en date du 01/03/93 l'utilisation par voie locale d'un sel d'acide hyaluronique mis au point par le Laboratoire FIDIA comme traitement symptomatique à effet prolongé de l'arthrose.

Par ailleurs, différents polydésoxyribonucléotides sont connus comme matières premières pharmaceutiques et utilisables également comme produits injectables et jouissent de ce fait d'une grande sécurité d'emploi.

La Demanderesse a maintenant découvert que l'utilisation de gels à base d'acide hyaluronique ou de polydésoxyribonucléotides comme matériaux de remplissage de prothèses conduisait à des implants qui ne présentaient aucun des inconvénients de ceux de l'art antérieur.

Par hydrogels à base d'acide hyaluronique et/ou de polydésoxyribonucléotides, au sens donc de l'invention, on entend des hydrogels biocompatibles préparés :
- à partir des biopolymères naturels d'acide hyaluronique et/ou de polydésoxyribonucléotides obtenus notamment par extraction ou fermentation, dénommés ci-après biopolymères naturels,
- à partir des biopolymères des dérivés d'acide hyaluronique et/ou de polydésoxyribonucléotides, en particulier des dérivés sous forme de sels ou d'esters, dénommés ci-après biopolymères dérivés,
- par réticulation des hydrogels de biopolymères naturels et dérivés définis ci-dessus,
- par réticulation de mélanges contenant des biopolymères naturels et/ou dérivés définis ci-dessus et un autre polymère.

Elle a également découvert que ces gels pouvaient être utilisés en mélange.

Elle a également découvert que des gels intéressants comme matériaux de remplissage étaient obtenus à partir de complexes de sels d'acide hyaluronique et de polydésoxyribonucléotides.

L'avantage particulier de l'invention est qu'elle offre une solution de rechange par rapport à l'utilisation classique, mais controversée à l'heure actuelle, des gels de silicone comme matériaux de remplissage des prothèses. Cette solution conduit à des prothèses présentant les mêmes avantages dans leur utilisation pour remplacer ou remodeler des tissus mous mais présentant, en outre, l'avantage de mettre en oeuvre des matériaux biocompatibles remplissant, en particulier, toutes les caractéristiques des recommandations de la norme NF EN 30993-1 concernant les essais de biocompatibilité des produits de remplissage.

Ainsi, selon l'une de ses caractéristiques essentielles, la présente invention concerne l'utilisation d'hydrogels à base d'acide hyaluronique et/ou de polydésoxyribonucléotides comme matériaux de remplissage de prothèses, en particulier de prothèses mammaires.

Selon une première variante, l'hydrogel est un hydrogel d'un sel d'acide hyaluronique.

Selon une deuxième variante de l'invention, l'hydrogel est constitué d'un hydrogel d'un polydésoxyribonucléotide.

Selon une troisième variante, l'hydrogel est constitué d'un mélange des deux types d'hydrogel ci-dessus.

L'acide hyaluronique peut être préparé par tout procédé connu d'extraction ou de fermentation.

Il peut être sous forme de polymère linéaire ou réticulé.

Lorsqu'on utilise un acide hyaluronique réticulé, on utilise de préférence un produit réticulé par un procédé physique de réticulation.

On peut également utiliser tout produit de réticulation conduisant à un hydrogel biocompatible.

Pour préparer les gels utilisables selon l'invention, on utilise avantageusement des dérivés d'acide hyaluronique correspondant à un acide hyaluronique présentant une masse moléculaire moyenne en poids comprise entre 10⁶ et 8.10⁶ daltons, de préférence entre 1 et 3.10⁶ daltons.

Parmi les sels d'acide hyaluronique préférés selon l'invention, on citera les sels d'acide hyaluronique, avec un cation, par exemple un sel mono- ou divalent tel que les sels de sodium, potassium, magnésium, calcium, manganèse.

Les sels de sodium sont tout particulièrement préférés. On part avantageusement, pour la préparation des gels utilisables selon l'invention, de solutions aqueuses de hyaluronate de sodium telles que la viscosité capillaire pour des solutions aqueuses à 0,01 % sur sec en poids est de préférence comprise entre 2,0.10⁻³ et 3,0.10⁻³ Pa.s (2,0 et 3,0 centipoises) à 30°C.

Selon une autre variante, les sels d'acide hyaluronique sont des sels d'acide hyaluronique et d'acide aminé naturel basique, par exemple des sels d'acide hyaluronique et de lysine, d'histidine ou d'arginine.

Tous les sels cités ci-dessus peuvent être des sels simples ou mixtes. Tous ces sels peuvent en particulier être fabriqués à partir d'acide hyaluronique de fermentation, la nature du sel dépendant des conditions de purification utilisées.

Selon une autre variante de l'invention, l'hydrogel est constitué d'un hydrogel d'un polydésoxyribonucléotide de masse molaire moyenne en poids comprise entre 1.10⁶ et 5.10⁶ daltons, de préférence entre 3.10⁶ et 4.10⁶ daltons. Les polydésoxyribonucléotides sont avantageusement des sels mono- ou divalents tels que les sels de sodium, potassium, magnésium, calcium, manganèse, avantageusement le sel de sodium.

Il peut également s'agir de sels de polydésoxyribonucléotides avec des acides aminés naturels basiques tels que la lysine, l'arginine.

Les polydésoxyribonucléotides peuvent être préparés par fermentation, extraction ou culture cellulaire. Ils sont avantageusement préparés par extraction à partir de laitance de poissons, en particulier de saumon. Suivant les conditions de purification, on prépare en particulier les différents sels simples cités ci-dessus ainsi qu'éventuellement des sels mixtes.

Les polydésoxyribonucléotides utilisables pour préparer les gels selon l'invention sont tels que la viscosité capillaire de leur solution aqueuse à 0,1 % sur sec est supérieure à 2.10⁻³ Pa.s (2 centipoises) à 30°C, de préférence comprise entre 2.10⁻³ et 3,5.10⁻³ Pa.s (2,0 et 3,5 centipoises).

Selon une autre variante de l'invention les hydrogels utilisables sont des mélanges des hydrogels d'acide hyaluronique et de polydésoxyribonucléotides définis précédemment.

Lorsque l'hydrogel est un hydrogel de sel d'acide hyaluronique, il contient 1 à 5 % en poids de sel d'acide hyaluronique, de préférence 2 à 4 %.

Lorsque l'hydrogel est un hydrogel de polydésoxyribonucléotide il contient de 2 à 4 % de polydésoxyribonucléotide.

Lorsque l'hydrogel est obtenu à partir d'un mélange de sel d'acide hyaluronique et de polydésoxyribonucléotide, la proportion de polydésoxyribonucléotide est avantageusement inférieure à 50 % en poids, de préférence comprise entre 1 et 10 %.

Selon une autre variante de l'invention, les hydrogels utilisables selon l'invention sont obtenus à partir de sels doubles de polydésoxyribonucléotides et d'acide hyaluronique. Ces sels sont obtenus à partir des sels d'acide hyaluronique et de polydésoxyribonucléotides définis précédemment.

La préparation de ces sels doubles consiste à ponter les chaînes d'acide hyaluronique et les chaînes d'acide désoxyribonucléique entre elles par des cations bivalents, de préférence du calcium ou du magnésium. La masse fibreuse recueillie après cette opération de pontage est un sel double des deux polymères finement enchevêtrés.

Les caractéristiques rhéologiques des gels obtenus à partir des sels doubles sont semblables à celles des deux polymères de départ et sont fonction des taux de mélange des deux polymères lors de la préparation. Les polydésoxyribonucléotides peuvent représenter jusqu'à 50 % du produit final. De préférence, ce taux sera compris entre 1 et 10 % en poids.

L'avantage que présente ce sel double dans la fabrication d'un gel est l'utilisation d'une matière première unique parfaitement définie et dosée dont les différents constituants sont déjà intimement mélangés.

Lorsque les hydrogels sont formés à partir des sels doubles ci-dessus, ils contiennent une proportion comprise entre 2 et 4 % en poids de ces polymères.

Les hydrogels utilisables selon l'invention sont préparés à partir des sels et mélanges définis précédemment et d'un milieu hydrophile biocompatible.

A titre de milieux hydrophiles biocompatibles, on citera l'eau pour préparation injectable, les solutions aqueuses isotoniques, en particulier des solutions à 0,9 % de NaCl, les solutions aqueuses isotoniques tamponnées.

Les hydrogels sont avantageusement préparés dans un mélangeur sous vide à double enveloppe permettant d'assurer leur dégazage et leur transfert par réduction de la viscosité.

Les hydrogels définis précédemment peuvent être également soumis à une réticulation destinée à créer des pontages entre les chaînes moléculaires, ce qui conduit à une augmentation de la viscosité desdits gels.

Cette réticulation peut être soit physique, soit chimique, le seul impératif étant que les produits obtenus soient biocompatibles.

Les pontages chimiques réalisés peuvent être par exemple de type éther, phosphate éther, amide ou sulfonate.

A titre d'exemples de composés chimiques utilisables pour créer un tel pontage, on citera le sulfone de divinyle, le formaldéhyde ainsi que les composés présentant une fonction époxyde.

Selon une autre variante de l'invention, la réticulation pourra être effectuée sur un mélange contenant majoritairement le biopolymère naturel ou dérivé tel qu'il a été décrit précédemment, ce biopolymère étant associé à un autre polymère, de préférence un polymère naturel.

A titre d'exemple de polymères avantageusement utlisés en association avec les biopolymères naturels ou dérivés utiles selon l'invention, on citera l'hydroxyméthylcellulose, le collagène, le chitosane, l'élastine.

Les gels obtenus à partir des chaînes polymériques linéaires ou réticulées sont ensuite utilisés soit pour remplir les enveloppes destinées à être utilisées comme prothèses dites prothèses pré-remplies, soit pour remplir des seringues destinées au remplissage des prothèses dites prothèses gonflables.

Ces prothèses pré-remplies ou les seringues sont ensuite soumises à une opération de stérilisation selon l'une des méthodes décrites par la pharmacopée, puis conditionnées stérilement.

Après stérilisation, la viscosité des gels mesurée à 37°C à l'aide d'un viscosimètre à mobile tournant (BROOKFIELD, LV-DV1, mobile n° 4) est comprise entre 10 et 300 Pa.s (10 000 et 300 000 centipoises) (v = 1,5 et 3,0 rpm), de préférence entre 50 et 200 Pa.s (50 000 et 200 000 centipoises) (v = 1,5 rpm) et entre 30 et 180 Pa.s (180 000 centipoises) (v = 3 rpm).

Les gels décrits précédemment sont utilisés selon l'invention pour remplir des enveloppes telles que celles utilisées à l'heure actuelle pour former des prothèses de type gonflables que l'on remplit donc du gel après implantation dans l'organisme ou de type pré-remplies que l'on remplit du gel avant implantation.

Le matériau utilisé pour la fabrication de telles enveloppes est généralement constitué d'élastomères de silicone, principalement d'élastomère de silicone vulcanisé à froid dans le cas des implants gonflables et d'élastomères de silicone réticulables à chaud dans le cas des implants pré-remplis. La forme et les dimensions de l'enveloppe dépendent bien entendu de la destination de l'implant.

D'une façon générale, on pourra remplir avec les gels décrits précédemment tout implant destiné au remplacement, à la substitution et l'augmentation d'un tissu mou. A titre d'exemple de tels implants, on citera les implants destinés à la reconstruction des mollets, des testicules et plus particulièrement les implants de reconstruction ou d'augmentation dans les indications de plasties mammaires (implants mammaires).

### Exemple

On remplit des prothèses mammaires gonflables de 60 ml avec différents gels d'acide hyaluronique dont la composition est donnée dans le tableau ci-dessous.

| Acide hyaluronique (% en poids) | Milieu hydrophile |
|---|---|
| 3 | Eau |
| 4 | Eau |
| 3 | NaCl 0,9 % (en poids) |
| 4 | NaCl 0,9 % (en poids) |
| 4 | NaCl 0,9 % - MgCl₂ 0,6 % (en poids) |
| 3 | Tampon phosphate |

Ces prothèses ont été soumises à un test de palpation par un chirurgien plasticien en comparaison avec une prothèse classique pré-remplie de gel de silicone et ont donné des résultats équivalents.

## Revendications

1. Utilisation d'hydrogels à base d'acide hyaluronique et/ou de polydésoxyribonucléotides comme materiaux de remplissage de prothèses, en particulier de prothèses mammaires.

2. Utilisation selon la revendication 1, caractérisée en ce que l'acide hyaluronique a une masse moléculaire moyenne en poids comprise entre 10⁶ et 8.10⁶ daltons.

3. Utilisation selon l'une des revendications 1 ou 2, caractérisée en ce que ledit acide hyaluronique est sous la forme d'un polymère linéaire.

4. Utilisation selon l'une des revendications 1 ou 2, caractérisée en ce que ledit acide hyaluronique est sous la forme d'un polymère réticulé.

5. Utilisation selon l'une des revendications 1 à 4, caractérisée en ce que le polydésoxyribonucléotide a une masse moléculaire moyenne en poids comprise entre 10⁶ et 5.10⁶ daltons.

6. Utilisation selon l'une des revendications 1 à 5, caractérisée en ce que l'hydrogel est un hydrogel d'un sel simple ou mixte, avec un cation mono- ou divalent ou avec un acide aminé naturel basique d'acide hyaluronique ou de désoxyribonucléotide

7. Utilisation selon la revendication 6, caractérisée en ce que ledit cation est le sodium, le magnésium, le potassium, le calcium, le manganèse, de préférence le sodium.

8. Utilisation selon l'une des revendications 1 à 7, caractérisée en ce que l'hydrogel est un hydrogel d'un sel d'acide hyaluronique contenant 1 à 5 % en poids de sel d'acide hyaluronique.

9. Utilisation selon la revendication 8, caractérisée en ce que l'hydrogel contient 2 à 4 % en poids de sel d'acide hyaluronique.

10. Utilisation selon l'une des revendications 1 à 7, caractérisée en ce que l'hydrogel est un hydrogel de polydésoxyribonucléotide contenant 2 à 4 % en poids de polydésoxyribonucléotide.

11. Utilisation selon l'une des revendications 1 à 7, caractérisée en ce que l'hydrogel est un mélange d'hydrogel de sel(s) d'acide hyaluronique et de sel(s) de polydésoxyribonucléotide, le(s) sel(s) de polydésoxyribonucléotide représentant au plus 50 % en poids du poids total des sel(s), de préférence 1 à 10 %.

12. Utilisation selon l'une des revendications 1 à 7, caractérisée en ce que l'hydrogel est un hydrogel d'un sel double d'acide hyaluronique et de polydésoxyribonucléotide.

13. Utilisation selon la revendication 12, caractérisée en ce que lesdits sels doubles sont obtenus par pontage entre les chaînes d'acide hyaluronique et d'acide polydésoxyribonucléique.

14. Utilisation selon l'une des revendications 12 ou 13, caractérisée en ce que ledit sel double contient jusqu'à 50 % en poids de polydésoxyribonucléotide, de préférence 1 à 10 %.

15. Utilisation selon l'une des revendications 12 à 14, caractérisée en ce que l'hydrogel comprend 2 à 4 % en poids dudit sel double.

16. Utilisation selon l'une des revendications 1 à 15, caractérisée en ce que l'hydrogel est soumis à une réticulation destinée à créer des pontages entre les chaînes moléculaires.

17. Utilisation selon l'une des revendications 1 à 16, caractérisée en ce que l'acide hyaluronique et/ou les polydésoxyribonucléotides et/ou leurs dérivés sont utilisés en mélange avec un autre polymère, de préférence un polymère naturel, ledit mélange contenant majoritairement l'acide hyaluronique et/ou les polydésoxyribonucléotides et/ou leurs dérivés.

18. Utilisation selon l'une des revendications 1 à 17, caractérisée en ce que la phase aqueuse constituant l'hydrogel est un mélange hydrophile biocompatible.

19. Utilisation selon l'une des revendications 1 à 18, caractérisée en ce que l'hydrogel présente une viscosité mesurée à 37°C à l'aide d'un viscosimètre à mobile tournant de type Brookfield LV-DV1, mobile n° 4, comprise entre 10 et 300 Pa.s (v = 1,5 et 3,0 rpm).

20. Prothèse comprenant une enveloppe remplie d'un hydrogel tel que défini dans les revendications 1 à 19.

## Claims

1. Use of hydrogels based on hyaluronic acid and/or polydeoxyribonucleotides as filling materials for prostheses, in particular mammary prostheses.

2. Use according to claim 1, characterised in that the hyaluronic acid has an average molecular mass by weight between 10⁶ and 8.10⁶ Daltons.

3. Use according to one of claims 1 or 2, characterised in that said hyaluronic acid is in the form of a linear polymer.

4. Use according to one of claims 1 or 2, characterised in that said hyaluronic acid is in the form of a cross-linked polymer.

5. Use according to one of claims 1 or 4, characterised in that the polydeoxyribonucleotide has an average molecular mass by weight between 10⁶ and 5.10⁶ Daltons.

6. Use according to one of claims 1 to 5, characterised in that the hydrogel is a hydrogel of a single or mixed salt of a mono- or divalent cation or of a basic natural amino acid with hyaluronic acid or with deoxyribonucleotide.

7. Use according to claim 6, characterised in that said cation is sodium, magnesium, potassium, calcium, manganese, preferably sodium.

8. Use according to one of claims 1 to 7, characterised in that the hydrogel is a hyaluronic acid salt hydrogel containing 1 to 5 % by weight of hyaluronic acid salt.

9. Use according to claim 8, characterised in that the hydrogel contains 2 to 4 % by weight of hyaluronic acid salt.

10. Use according to one of claims 1 to 7, characterised in that the hydrogel is a polydeoxyribonucleotide hydrogel containing 2 to 4 % by weight of polydeoxyribonucleotide.

11. Use according to one of claims 1 to 7, characterised in that the hydrogel is a mixture of hydrogel of hyaluronic acid salt(s) and of polydeoxyribonucleotide salt(s), the polydeoxyribonucleotide salt(s) representing at the most 50 % by weight of the total weight of the salt(s), preferably 1 to 10 %.

12. Use according to one of claims 1 to 7, characterised in that the hydrogel is a hydrogel of a double salt of hyaluronic acid and polydeoxyribonucleotide.

13. Use according to claim 12, characterised in that said double salts are obtained by bridging between the chains of hyaluronic acid and polydeoxyribonucleic acid.

14. Use according to one of claims 12 or 13, characterised in that said double salt contains up to 50 % by weight of polydeoxyribonucleotide, preferably 1 to 10 %.

15. Use according to one of claims 12 to 14, characterised in that the hydrogel comprises 2 to 4 % by weight of said double salt.

16. Use according to one of claims 1 to 15, characterised in that the hydrogel is subjected to a cross-linking for creating bridges between the molecular chains.

17. Use according to one of claims 1 to 16, characterised in that the hyaluronic acid and/or the polydeoxyribonucleotides and/or their derivatives are used in a mixture with another polymer, preferably a natural polymer, said mixture containing for the most part the hyaluronic acid and/or the polydeoxyribonucleotides and/or their derivatives.

18. Use according to one of claims 1 to 17, characterised in that the aqueous phase constituting the hydrogel is a biocompatible hydrophilic mixture.

19. Use according to one of claims 1 to 18, characterised in that the hydrogel has a viscosity measured at 37°C with a viscometer having a rotary body of the type Brookfield LV-DV1, body No. 4, between 10 and 300 Pa.s (v = 1.5 and 3.0 rpm).

20. A prosthesis comprising an envelope filled with a hydrogel such as defined in claims 1 to 19.

## Patentansprüche

1. Verwendung von Hydrogelen auf Basis von Hyaluronsäure und/oder Polydesoxyribonucleotiden als Füllmaterial für Prothesen, insbesondere Mamma-Prothesen.

2. Verwendung nach Anspruch 1, dadurch gekennzeichnet, daß die Hyaluronsäure eine gewichtsdurchschnittliche Molekularmasse zwischen 10⁶ und 8.10⁶ Dalton aufweist.

3. Verwendung nach einem der Ansprüche 1 oder 2, dadurch gekennzeichnet, daß die genannte Hyaluronsäure in Form eines linearen Polymers vorliegt.

4. Verwendung nach einem der Ansprüche 1 oder 2, dadurch gekennzeichnet, daß die genannte Hyaluronsäure in Form eines vernetzten Polymers vorliegt.

5. Verwendung nach einem der Ansprüche 1 bis 4, dadurch gekennzeichnet, daß das Polydesoxyribonucleotid eine gewichtsdurchschnittliche Molekularmasse zwischen 10⁶ und 5.10⁶ Dalton aufweist.

6. Verwendung nach einem der Ansprüche 1 bis 5, dadurch gekennzeichnet, daß das Hydrogel ein Hydrogel aus einem einfachen oder gemischten Hyaluronsäure- oder Desoxyribonucleotid-Salz ist mit einem mono- oder divalenten Kation oder mit einer natürlichen basischen Aminosäure.

7. Verwendung nach Anspruch 6, dadurch gekennzeichnet, daß das genannte Kation Natrium, Magnesium, Kalium, Calcium oder Mangan, vorzugsweise Natrium, ist.

8. Verwendung nach einem der Ansprüche 1 bis 7, dadurch gekennzeichnet, daß das Hydrogel ein Hyaluronsäure-Salz-Hydrogel ist, das 1 bis 5 Gew.-% Hyaluronsäuresalz enthält.

9. Verwendung nach Anspruch 8, dadurch gekennzeichnet, daß das Hydrogel 2 bis 4 Gew.-% Hyaluronsäuresalz enthält.

10. Verwendung nach einem der Ansprüche 1 bis 7, dadurch gekennzeichnet, daß das Hydrogel ein Polydesoxyribonucleotid-Hydrogel ist, das 2 bis 4 Gew.-% Polydesoxyribonucleotid enthält.

11. Verwendung nach einem der Ansprüche 1 bis 7, dadurch gekennzeichnet, daß das Hydrogel ein Hydrogelgemisch von einem oder mehreren Hyaluronsäuresalzen und einem oder mehreren Polydesoxyribonucleotid-Salzen ist, wobei das (die) Polydesoxyribonucleotidsalz(e) höchstens 50 Gew.-%, vorzugsweise 1 bis 10 Gew.-%, des Gesamtgewichts des (der) Salze(s) darstellt (darstellen).

12. Verwendung nach einem der Ansprüche 1 bis 7, dadurch gekennzeichnet, daß das Hydrogel ein Hydrogel eines Hyaluronsäure/Polydesoxyribonucleotid-Doppelsalzes ist.

13. Verwendung nach Anspruch 12, dadurch gekennzeichnet, daß die genannten Doppelsalze durch Brückenbindung zwischen den Ketten der Hyaluronsäure und der Polydesoxyribonucleinsäure erhalten wurden.

14. Verwendung nach einem der Ansprüche 12 oder 13, dadurch gekennzeichnet, daß das genannte Doppelsalz bis zu 50 Gew.-%, vorzugsweise 1 bis 10 Gew.-%, Polydesoxyribonucleotid enthält.

15. Verwendung nach einem der Ansprüche 12 bis 14, dadurch gekennzeichnet, daß das Hydrogel 2 bis 4 Gew.-% des genannten Doppelsalzes umfaßt.

16. Verwendung nach einem der Ansprüche 1 bis 15, dadurch gekennzeichnet, daß das Hydrogel einer Vernetzung unterworfen wurde zur Erzeugung von Brückenbindungen zwischen den Molekülketten.

17. Verwendung nach einem der Ansprüche 1 bis 16, dadurch gekennzeichnet, daß die Hyaluronsäure und/oder die Polydesoxyribonucleotide und/oder ihre Derivate im Gemisch mit einem anderen Polymer, vorzugsweise einem natürlichen Polymer, verwendet werden, wobei das genannte Gemisch überwiegend die Hyaluronsäure und/oder die Polydesoxyribonucleotide und/oder ihre Derivate enthält.

18. Verwendung nach einem der Ansprüche 1 bis 17, dadurch gekennzeichnet, daß die wäßrige Phase, die das Hydrogel darstellt, ein biokompatibles hydrophiles Gemisch ist.

19. Verwendung nach einem der Ansprüche 1 bis 18, dadurch gekennzeichnet, daß das Hydrogel eine Viskosität zwischen 10 und 300 Pa.s aufweist, gemessen bei 37°C unter Verwendung eines Viskosimeters mit beweglicher Spindel vom Brookfield LV-DV1-Typ, Spindel Nr. 4 (v = 1,5 und 3,0 UpM).

20. Prothese, die eine Hülle umfaßt, die mit einem Hydrogel, wie es in den Ansprüchen 1 bis 19 definiert ist, gefüllt ist.
